(19) Europäisches Patentamt
European Patent Office
Office européen des brevets

(11) EP 0 709 361 B1

(12) **FASCICULE DE BREVET EUROPEEN**

(45) Date de publication et mention de la délivrance du brevet:
**03.02.1999 Bulletin 1999/05**

(51) Int. Cl.$^6$: **C07C 45/67**, C07C 45/71, C07C 47/575

(21) Numéro de dépôt: **95402336.2**

(22) Date de dépôt: **20.10.1995**

(54) **Procédé de préparation d'isovanilline**

Verfahren zur Herstellung von Isovanillin

Process for the preparation of isovanilline

(84) Etats contractants désignés:
**AT BE CH DE DK ES FR GB IE IT LI NL PT SE**

(30) Priorité: **24.10.1994 FR 9412661**

(43) Date de publication de la demande:
**01.05.1996 Bulletin 1996/18**

(73) Titulaire: **RHODIA CHIMIE**
**92408 Courbevoie Cédex (FR)**

(72) Inventeur: **Maliverney, Christian**
**F-69007 Lyon (FR)**

(74) Mandataire:
**Dutruc-Rosset, Marie-Claude et al**
**RHODIA SERVICES**
**Direction de la Propriété Industrielle**
**25, quai Paul Doumer**
**92408 Courbevoie Cédex (FR)**

(56) Documents cités:
**DE-C- 622 966**
**US-A- 3 007 968**
**US-A- 3 367 972**
**US-A- 4 065 504**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 709 361 B1

Printed by Xerox (UK) Business Services
2.16.7/3.6

## Description

La présente invention a pour objet un nouveau procédé de préparation d'isovanilline.

L'isovanilline ou 3-hydroxy-4-méthoxybenzaldéhyde est un intermédiaire de synthèse utilisé notamment dans le domaine pharmaceutique, cosmétique, agrochimique et alimentaire.

Plusieurs voies d'accès sont décrites dans l'état de la technique et l'on peut mentionner en particulier les procédés qui consistent à partir de l'héliotropine (ou 3,4-(méthylènedioxy)benzaldéhyde) et à la faire réagir avec le méthylate de sodium, en présence de chlorure cuivreux, dans un solvant organique, à savoir le diméthylformamide [Baratov et al, Zh. Org. Khim., 27, (7), 1578 (1991)]. Les inconvénients majeurs de ce procédé sont le coût du substrat de départ utilisé ainsi que ceux liés à la mise en oeuvre d'un solvant organique qu'il faut récupérer afin de le recycler.

L'isovanilline peut être également préparée à partir du vératraldéhyde (ou 3,4-diméthoxybenzaldéhyde) en effectuant une déméthylation sélective à l'aide de méthionine dans l'acide méthanesulfonique [Fujii et al, J. Chem. Soc. Perkin. Trans I, 20, 2288 (1977)]. Le rocédé est long, l'acide méthanesulfonique coûteux et la sélectivité en isovanilline pas très bonne.

Il est également décrit par Kessar et al [J. Chem. Soc. Chem. Commun. 7, 400 (1983)], un procédé d'obtention d'isovanilline par O-méthylation sélective de l'aldéhyde protocatéchique (ou 3,4-dihydroxybenzaldéhyde) par l'iodure de méthyle, en présence d'hydrure de sodium et dans le diméthysulfoxyde. Le rendement en isovanilline obtenu n'est que de 65 %. Cette voie de synthèse souffre également d'une absence de compétitivité d'un point de vue économique car la matière première de départ n'est pas un produit industriel et la présence du solvant organique accroît le coût de production.

Un autre procédé également coûteux décrit par Scarpati et al, [Synth. Com.20, (17), 2565 (1990)] réside dans la formylation du gaïacol protégé sous la forme d'acétate, par le dichlorométhoxyméthane, en présence de tétrachlorure de titane, dans le dichlorométhane ce qui conduit au 3-acétoxy-4-méthoxybenzaldéhyde qui est ensuite hydrolysé à l'aide de soude. Outre le nombre d'étapes élevé, ce procédé fait appel au dichlorométhoxyméthane qui est un produit cher et toxique et fait intervenir également un solvant organique.

Par ailleurs, on a décrit dans US-A-3 367 972, un procédé de préparation d'isovanilline par hydrolyse acide de l'aldéhyde vératrique. Toutefois, la réaction est lente et pas très sélective. Dans l'exemple 1, le taux de transformation de l'aldéhyde n'est que de 35,8 %, au bout de 360 mn à 70,5-72,3°C et l'on obtient de l'isovanilline (33,2 %) accompagnée de vanilline (2,9 %) qu'il faut séparer.

Tous ces procédés ne donnent pas satisfaction car ils sont difficilement transposables à l'échelle industrielle, soit en raison de mauvais rendements réactionnels, soit pour des raisons économiques.

L'objet de la présente invention est de fournir un procédé perfectionné permettant d'obvier aux inconvénients précités.

Il a maintenant été trouvé que l'on pouvait préparer l'isovanilline, selon une réaction rapide et avec un très bon rendement réactionnel selon un procédé qui consiste à effectuer la désalkylation à l'aide d'un acide fort, sélectivement en position 3, d'un 3-alkoxy-4-méthoxybenzaldéhyde dans lequel le groupe alkoxy a au moins deux atomes de carbone.

En effet, on a mis en évidence que l'isovanilline pouvait être préparée dans des conditions économiques intéressantes dans la mesure où l'on effectuait la désalkylation d'un 3-alkoxy-4-méthoxybenzaldéhyde dont le groupe alkoxy partant présente une condensation en carbone supérieure au groupe méthoxy.

Selon un mode préféré de réalisation de l'invention, on effectue la préparation du 3-alkoxy-4-méthoxybenzaldéhyde en effectuant la O-méthylation d'un 3-alkoxy-4-hydroxybenzaldéhyde présentant un groupe alkoxy ayant au moins deux atomes de carbone.

Ainsi, dans sa forme préférée, le procédé de préparation d'isovanilline, selon l'invention, comprend :

- une première étape, où l'on effectue la O-méthylation d'un 3-alkoxy-4-hydroxybenzaldéhyde présentant un groupe alkoxy ayant au moins deux atomes de carbone,
- dans une deuxième étape, où l'on effectue la désalkylation sélective en position 3 du 3-alkoxy-4-méthoxybenzaldéhyde obtenu, avec un acide fort permettant ainsi d'obtenir l'isovanilline.

Conformément au procédé de l'invention, on part de 3-alkoxy-4-méthoxybenzaldéhyde répondant plus particulièrement à la formule (I) suivante :

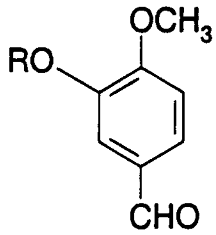

(I)

dans ladite formule (I), R représente un radical alkyle ou cycloalkyle ayant au moins 2 atomes de carbone.

L'invention fait intervenir préférentiellement un 3-alkoxy-4-méthoxy-benzaldéhyde de formule (I) dans laquelle le radical R est préférentiellement un radical alkyle ou cycloalkyle dont le nombre d'atomes de carbone varie entre 2 et 8 atomes de carbone.

Si d'un point de vue chimique, la nature du radical choisi importe peu dans la mesure où il comprend au moins deux atomes de carbone, il est à noter que d'un point de vue économique, il est préférable de choisir le radical R parmi les radicaux éthyle, propyle, isopropyle, n-butyle, isobutyle, sec-butyle, tert-butyle.

Parmi les substrats de formule (I), on met en oeuvre tout particulièrement, le 3-éthoxy-4-méthoxybenzaldéhyde, le 3-n-propoxy-4-méthoxybenzaldéhyde, le 3-isopropoxy-4-méthoxybenzaldéhyde, le 3-n-butoxy-4-méthoxybenzaldéhyde, le 3-isobutoxy-4-méthoxybenzaldéhyde, le 3-sec-butoxy-4-méthoxybenzaldéhyde, le 3-tert-butoxy-4-méthoxybenzaldéhyde.

Conformément au procédé de l'invention, on fait réagir le 3-alkoxy-4-méthoxybenzaldéhyde avec un acide fort.

Plusieurs impératifs président au choix du solvant organique. Une première caractéristique de cet acide, est que ce soit un acide fort. Par acide fort, on désigne un acide présentant un pKa dans l'eau inférieur à 3.

La borne inférieure ne présente aucun caractère critique.

Le pKa est défini comme la constante de dissociation ionique du couple acide/base, lorsque l'eau est utilisée comme solvant.

Une deuxième caractéristique de cet acide, est que ce soit un acide non oxydant dans les conditions de la réaction. Ainsi, sont donc exclus de l'invention, des acides tels que l'acide nitrique.

Comme d'exemples d'acides convenant particulièrement bien à la mise en oeuvre du procédé de l'invention, on peut citer les acides halogénés tels que l'acide chlorhydrique, l'acide bromhydrique, l'acide iodohydrique.

Il est également possible de faire appel à des oxyacides halogénés ou non tels que l'acide sulfurique, l'acide pyrosulfurique ; les acides sulfoniques halogénés ou non tels que l'acide fluorosulfonique, l'acide chlorosulfonique, l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence les acides chlorhydrique, bromhydrique et acide sulfurique.

L'acide sulfurique est choisi préférentiellement.

La concentration de l'acide fort de départ peut varier en fonction des disponibilités du commerce.

Par exemple, si l'on fait appel à l'acide chlorhydrique ou à l'acide bromhydrique, la concentration de l'acide varie entre 30 et 50 % en poids.

Dans le cas où l'on utilise l'acide sulfurique, la concentration de celui-ci varie avantageusement entre 80 et 100 % en poids, de préférence entre 90 et 99 % en poids. Selon un mode préférentiel de réalisation de l'invention, on fait appel à une solution d'acide sulfurique, la plus concentrée possible : la concentration se situant entre 95 et 99 % en poids.

Le rapport entre le nombre de moles d'acide fort mises en oeuvre et le nombre de moles de 3-alkoxy-4-méthoxybenzaldéhyde, peut varier largement, par exemple entre 2 et 80.

Il est préférable que ledit rapport molaire se situe entre 3 et 15 et encore plus préférentiellement entre 4 et 10.

La température à laquelle est conduit le procédé de l'invention peut varier, largement, par exemple entre 0°C et 150°C.

D'un point de vue économique, il y a intérêt à ne pas chauffer le milieu réactionnel. Toutefois, un chauffage peut s'avérer nécessaire dans certains cas.

La température choisie est fonction de la nature de l'acide utilisé et de la nature du groupe R à libérer.

On a trouvé que la température réactionnelle peut être choisie d'autant plus basse que le groupe R à libérer est gros et ramifié.

La température réactionnelle adéquate à mettre en oeuvre est aisément déterminable par l'Homme du Métier par la réalisation de simples opérations d'exécution.

Ainsi, on donne ci-après des exemples illustrant la gamme de températures choisie.

Lorsque l'on met en oeuvre l'acide sulfurique, la température varie généralement entre 0°C et 100°C.

Lorsque le groupe R à libérer est un groupe alkyle linéaire tel que par exemple, éthyle, n-propyle, n-butyle, la température de la réaction est choisie avantageusement entre 50 et 90°C, de préférence, entre 60°C et 80°C.

Dans le cas où R est un groupe alkyle ramifié c'est-à-dire un groupe dans lequel l'atome de carbone situé en position a par rapport à l'atome d'oxygène est au moins secondaire, tel qu'un groupe isopropyle, sec-butyle, tert-butyle, la température de la réaction est comprise de préférence, entre 0°C et 40°C, et encore plus préférentiellement entre 0°C et 30°C.

Si l'on fait appel à un acide halogéné tel que l'acide chlorhydrique ou l'acide bromhydrique, la température de la réaction est choisie dans le cas d'un groupe R alkyle linéaire, de préférence, entre 100°C et 150°C, et encore plus préférentiellement entre 100°C et 130°C.

Si le groupe R est un groupe alkyle ramifié, la température de la réaction est alors choisie avantageusement entre 50 et 100°C, de préférence, entre 60°C et 80°C.

D'un point de vue pratique, le procédé de l'invention est facile à mettre en oeuvre car il ne nécessite pas d'avoir recours à un appareillage spécifique.

Pratiquement, le procédé de l'invention peut être mis en oeuvre de la manière décrite ci-après.

On charge les différents constituants du mélange réactionnel dans l'appareillage choisi. D'une manière préférée, on introduit l'acide fort dans le 3-alkoxy-4-méthoxybenzaldéhyde mais il est également possible d'effectuer l'opération inverse. Ainsi, on peut introduire le 3-alkoxy-4-méthoxybenzaldéhyde fondu dans l'acide fort ou ajouter le substrat en suspension dans l'acide fort.

Il est préférable de conduire le procédé de l'invention sous une atmosphère de gaz secs. On peut établir une atmosphère de gaz rares, de préférence l'argon mais l'on préfère l'azote.

Une fois l'atmosphère de gaz sec établie, on amène le mélange réactionnel à la température choisie, éventuellement par chauffage.

La durée de la réaction peut varier largement. Elle est dépendante de la nature du 3-alkoxy-4-méthoxybenzaldéhyde, de l'acide fort et de sa concentration ainsi que de la température.

Dans les conditions préférées de l'invention qui sont le recours à un acide fort concentré, la durée de la réaction varie par exemple de 30 minutes à 6 heures, selon la température à laquelle on opère.

En fin de réaction, la masse réactionnelle est ramenée à la température ambiante. Par température ambiante, on entend généralement une température comprise entre 18°C et 25 °C.

Dans certains cas, il se peut que l'on soit obligé de refroidir à une température comprise entre 10°C et 15°C.

On donne ci-après, à titre illustratif, un mode d'exécution permettant de séparer le produit recherché à partir de la masse réactionnelle.

Généralement, en raison de la présence d'acide fort concentré, on dilue la masse réactionnelle avec de l'eau ou de la glace de telle sorte que la concentration en acide fort soit de 20 à 30 % en poids.

On extrait l'isovanilline formée par un solvant organique insoluble dans l'eau et l'on peut citer notamment les éthers-oxydes, de préférence, l'éther éthylique ou isopropylique ; les hydrocarbures chlorés, de préférence, le chloroforme, le dichloroéthane ; les cétones insolubles dans l'eau, de préférence, la méthylisobutylcétone.

On choisit préférentiellement la méthylisoburylcétone.

Les phases organique et aqueuse sont séparées.

La phase aqueuse contient l'acide fort qui peut être régénéré et recyclé.

La phase organique est lavée à l'eau puis l'on ajoute un agent basique de telle sorte que le pH soit compris entre 6,5 et 7,0.

On fait appel de préférence à une solution aqueuse de soude.

On évapore le solvant organique. L'isovanilline précipite. On la sépare selon les techniques classiques de séparation solide/liquide, de préférence par filtration.

Le solide obtenu peut être purifié, notamment par cristallisation.

Selon un mode préféré de réalisation de l'invention, on effectue la préparation du 3-alkoxy-4-méthoxybenzaldéhyde en effectuant la O-méthylation d'un 3-alkoxy-4-hydroxybenzaldéhyde présentant un groupe alkoxy ayant au moins deux atomes de carbone.

Conformément à un mode de réalisation préféré du procédé de l'invention, on part de 3-alkoxy-4-hydroxybenzaldéhyde répondant plus particulièrement à la formule (II) suivante :

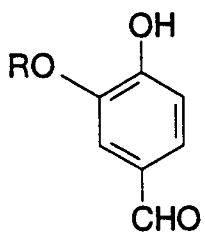

(II)

dans ladite formule (II), R représente un radical alkyle ou cycloalkyle ayant au moins 2 atomes de carbone.

L'invention fait intervenir de préférence un 3-alkoxy-4-hydroxybenzaldéhyde de formule (II) dans laquelle le radical R est préférentiellement, un radical alkyle ou cycloalkyle dont le nombre d'atomes de carbone peut varier entre 2 et 8 atomes de carbone.

Parmi les substrats de formule (II), on met en oeuvre tout particulièrement, le 3-éthoxy-4-hydroxybenzaldéhyde, le 3-n-propoxy-4-hydroxybenzaldéhyde, le 3-isopropoxy-4-hydroxybenzaldéhyde, le 3-n-butoxy-4-hydroxybenzaldéhyde, le 3-isobutoxy-4-hydroxybenzaldéhyde, le 3-sec-butoxy-4-hydroxybenzaldéhyde, le 3-tert-butoxy-4-hydroxybenzaldéhyde.

Dans le cas où l'on fait appel au 3-éthoxy-4-hydroxybenzaldéhyde qui est un produit commercialement disponible sur le marché, le procédé de l'invention est très compétitif vis-à-vis des procédés décrits.

Selon l'invention, on effectue la O-méthylation du 3-alkoxy-4-hydroxybenzaldéhyde, en le faisant réagir avec un agent de méthylation.

On peut faire appel à différents types d'agents alkylants et l'on peut citer plus particulièrement, les halogénures de méthyle, de préférence le chlorure de méthyle, le bromure de méthyle, le diméthylsulfate, le p-toluènesulfonate de méthyle, le méthanesulfonate de méthyle.

Parmi les agents alkylants précités, le chlorure de méthyle et le diméthylsulfate sont préférés.

Généralement, la réaction de O-méthylation est avantageusement conduite à un pH compris entre 8 et 10 et de préférence, supérieur à 9.

L'obtention du pH précité peut être si nécessaire assurée par addition d'une base, de préférence, une solution aqueuse d'un hydroxyde de métal alcalin ou de carbonate ou d'hydrogénocarbonate de métal alcalin. Tous les hydroxyde, carbonate ou hydrogénocarbonate de métaux alcalins peuvent être utilisés. La concentration de la solution de l'agent basique est choisie avantageusement élevée, de préférence entre 30 et 60 % en poids.

Cependant pour des considérations économiques, la soude est préférée et l'on fait appel plus préférentiellement aux solutions commerciales de soude dont la concentration est de 30 %.

Il n'y a aucun inconvénient à utiliser une base organique mais d'un point de vue économique, la soude est choisie préférentiellement.

Pour ce qui est de la quantité des réactifs à mettre en oeuvre dans cette étape de O-alkylation, on définit ci-après les quantités préférées.

La concentration du substrat de départ, à savoir le 3-alkoxy-4-hydroxy-benzaldéhyde dans le milieu réactionnel est avantageusement comprise entre 10 et 25 %.

La quantité d'agent d'alkylation engagée est fonction de la quantité de substrat à alkoxyler. Elle est de préférence au moins égale à la quantité stoechiométrique jusqu'à un excès pouvant atteindre 100 %.

En d'autres termes, le rapport molaire agent d'alkylation/3-alkoxy-4-hydroxy-benzaldéhyde varie entre 1,0 à 2,0 et, de préférence, entre 1,1 et 1,5.

En ce qui concerne les conditions réactionnelles, il est préférable de conduire le procédé de l'invention sous une atmosphère de gaz inerte. On peut établir une atmosphère de gaz rares, de préférence l'argon mais il est plus économique de faire appel à l'azote.

Une fois l'atmosphère de gaz inerte établie, on amène le mélange réactionnel à la température choisie.

La température de la réaction de O-méthylation n'est pas critique ; elle a une influence sur la cinétique de la réaction. Généralement, on réalise la réaction de O-méthylation entre 60°C et 100°C. De préférence, on opère à une température entre 80°C et 95°C.

La durée de la réaction de O-méthylation est fonction notamment de la température réactionnelle. Elle varie le plus souvent entre 1 heure et 8 heures. Généralement, une durée de 1 à 4 heures est suffisante.

Selon un mode de réalisation pratique de l'invention, on peut introduire d'abord le 3-alkoxy-4-hydroxybenzaldéhyde, la solution basique, amener le mélange à la température réactionnelle, puis ajouter l'agent d'alkylation et éventuellement la base si celle-ci est nécessaire pour ajuster le pH dans la zone précitée.

En fin de réaction, après refroidissement, on récupère une phase organique comprenant le 3-alkoxy-4-méthoxybenzaldéhyde.

Il est possible d'engager la phase organique, sans aucune séparation, à l'étape de désalkylation après élimination préférentielle de l'eau.

Il est également possible de séparer le 3-alkoxy-4-méthoxybenzaldéhyde, selon les techniques classiques de séparation.

On peut en particulier le séparer par extraction dans un solvant approprié, insoluble dans l'eau. Les solvants cités précédemment conviennent mais l'on choisit préférentiellement la méthylisobutylcétone.

Le 3-alkoxy-4-méthoxybenzaldéhyde obtenu peut être éventuellement purifié d'une manière classique, par distillation ou par cristallisation.

Il est ensuite engagé dans l'étape de désalkylation, selon le procédé de l'invention.

Le procédé de l'invention permet d'obtenir sélectivement l'isovanilline.

Les exemples qui suivent illustrent l'invention.

EXEMPLES

Dans les exemples, les abréviations ont la signification suivante :

- EVA = éthylvanilline = 3-éthoxy-4-hydroxybenzaldéhyde,
- EMBA = 3-éthoxy-4-méthoxybenzaldéhyde,
-

$$\text{Rendement} = \frac{\text{nombre de moles d'isovanilline formées}}{\text{nombre de moles de EMBA engagées}} \%$$

Exemple 1

Méthylation de l'éthylvanilline par le diméthylsulfate

Dans un ballon tricol de 2 000 ml, chauffé par chauffe-ballon électrique, agité mécaniquement, équipé de deux ampoules de coulée, d'un réfrigérant, de dispositifs de mesure de température et de pH, d'une arrivée d'azote, on charge 332,4 g d'éthylvanilline, après avoir établi une atmosphère d'azote.

On ajoute 0,5 litre d'eau et l'on chauffe à 90°C.

On ajoute 360 g d'une solution aqueuse de soude à 30,5 % (pH = 9), puis 350 g de diméthylsulfate en 2 heures.

On laisse le milieu réactionnel, 1 heure à 90°C sous agitation, puis l'on refroidit à 70°C et on laisse décanter.

On lave la couche supérieure organique qui est ensuite distillée à 120°C sous pression réduite de 10 mm de mercure (1330 Pa), pour donner le 3-éthoxy-4-méthoxy-benzaldéhyde.

Les résultats obtenus, après dosage par chromatographie liquide haute performance sont les suivants :

- Rendement avant distillation
: 98,4 %
- Pureté avant distillation
: 97 % (3 % d'eau)
- Pureté après distillation
: 99,9 %

Déséthylation du 3-éthoxy-4-méthoxybenzaldéhyde par l'acide sulfurique

Dans un réacteur à double enveloppe de 1,5 litre, équipé d'une agitation mécanique, d'un thermomètre et d'une arrivée d'azote, on charge 270,5 g de 3-éthoxy-4-méthoxybenzaldéhyde, 742 g d'acide sulfurique à 98 %, après avoir établi l'atmosphère de gaz inerte.

On chauffe 3 heures 30 minutes, à 65°C.

Le dosage par chromatographie liquide haute performance donne :

- Conversion de l'EMBA = 98,5 %
- Rendement en isovanilline = 96 %

On refroidit la masse réactionnelle qui est coulée dans 2 litres d'eau glacée sous agitation.
Le précipité qui se forme est dissous avec 2 litres de méthylisobutylcétone.
On sépare la phase organique ; la phase aqueuse réextraite avec 0,75 litre de méthylisobutylcétone. Les eaux-mères ($H_2SO_4$ à 30 %) peuvent être régénérées.
On lave la phase organique à pH = 7, puis on l'évapore à 60°C sous pression réduite d'environ 100 mm de mercure (13300 Pa) pour donner l'isovanilline (214 g) de pureté égale à 95 %.

## Revendications

1. Procédé de préparation d'isovanilline caractérisé par le fait qu'il consiste à effectuer la désalkylation à l'aide d'un acide fort, sélectivement en position 3, d'un 3-alkoxy-4-méthoxybenzaldéhyde dans lequel le groupe alkoxy a au moins deux atomes de carbone.

2. Procédé selon la revendication 1 caractérisé par le fait que le 3-alkoxy-4-méthoxybenzaldéhyde répond à la formule (I) suivante :

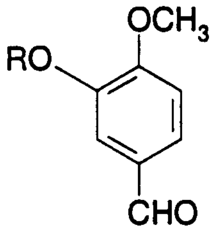
(I)

dans ladite formule (I), R représente un radical alkyle ou cycloalkyle ayant au moins 2 atomes de carbone.

3. Procédé selon l'une des revendications 1 et 2 caractérisé par le fait que le 3-alkoxy-4-méthoxybenzaldéhyde répond à la formule (I) dans laquelle le radical R est un radical alkyle ou cycloalkyle dont le nombre d'atomes de carbone varie entre 2 et 8 atomes de carbone et plus préférentiellement un radical éthyle, propyle, isopropyle, n-butyle, isobutyle, sec-butyle, tert-butyle.

4. Procédé selon l'une des revendications 1 à 3 caractérisé par le fait que le 3-alkoxy-4-méthoxybenzaldéhyde est le 3-éthoxy-4-méthoxybenzaldéhyde, le 3-n-propoxy-4-méthoxybenzaldéhyde, le 3-isopropoxy-4-méthoxybenzaldéhyde, le 3-n-butoxy-4-méthoxybenzaldéhyde, le 3-isobutoxy-4-méthoxybenzaldéhyde, le 3-sec-butoxy-4-méthoxybenzaldéhyde, le 3-tert-butoxy-4-méthoxybenzaldéhyde.

5. Procédé selon l'une des revendications 1 à 4 caractérisé par le fait que l'acide fort présente un pKa inférieur ou égal à 3.

6. Procédé selon la revendication 5 caractérisé par le fait que l'acide fort mis en oeuvre est choisi parmi : les acides halogénés, tels que l'acide chlorhydrique, l'acide bromhydrique, l'acide iodohydrique ; les oxyacides halogénés ou non, de préférence l'acide sulfurique, l'acide pyrosulfurique, l'acide perchlorique ; les acides sulfoniques halogénés ou non, de préférence l'acide fluorosulfonique, l'acide chlorosulfonique, l'acide trifluorométhanesulfonique, l'acide méthanesulfonique, l'acide éthanesulfonique, l'acide éthanedisulfonique, l'acide benzène-sulfonique, les acides benzènedisulfoniques, les acides toluènesulfoniques, les acides naphtalènesulfoniques et les acides naphtalènedisulfoniques.

7. Procédé selon l'une des revendications 5 et 6 caractérisé par le fait que l'acide fort mis en oeuvre est l'acide chlorhydrique, l'acide bromhydrique, ou l'acide sulfurique.

8. Procédé selon l'une des revendications 5 à 7 caractérisé par le fait que la concentration en acide fort varie entre 30 et 50 % en poids lorsque l'acide fort est l'acide chlorhydrique ou l'acide bromhydrique.

9. Procédé selon l'une des revendications 5 à 8 caractérisé par le fait que la concentration en acide fort varie entre

80 et 100 % en poids, de préférence entre 90 et 99 % en poids, et encore plus préférentiellement entre 95 et 99 % en poids, lorsque l'acide fort est l'acide sulfurique.

**10.** Procédé selon l'une des revendications 5 à 9 caractérisé par le fait que le rapport entre le nombre de moles d'acide fort mises en oeuvre et le nombre de moles de 3-alkoxy-4-méthoxybenzaldéhyde varie entre 2 et 80, de préférence entre 3 et 15, et encore plus préférentiellement entre 4 et 10.

**11.** Procédé selon l'une des revendications 1 à 10 caractérisé par le fait que la température de la réaction varie entre 0°C et 150°C.

**12.** Procédé selon la revendication 11 caractérisé par le fait que la température de la réaction varie : dans le cas de l'acide sulfurique, entre 50 et 90°C, de préférence, entre 60°C et 80°C lorsque le groupe alkyle à libérer est un groupe alkyle linéaire et entre 0°C et 40°C, de préférence, entre 0°C et 30°C lorsqu'il s'agit d'un groupe alkyle ramifié ; dans le cas de l'acide chlorhydrique ou l'acide bromhydrique entre 100°C et 150°C, de préférence, entre100°C et 130°C lorsque le groupe alkyle à libérer est un groupe alkyle linéaire et entre 50°C et 100°C, de préférence, entre 60°C et 80°C lorsqu'il s'agit d'un groupe alkyle ramifié.

**13.** Procédé selon l'une des revendications 1 à 12 caractérisé par le fait que l'on introduit la solution d'acide fort dans le 3-alkoxy-4-méthoxybenzaldéhyde ou inversement.

**14.** Procédé selon l'une des revendications 1 à 13 caractérisé par le fait que l'on introduit le 3-alkoxy-4-méthoxybenzaldéhyde fondu dans l'acide fort ou l'on ajoute le substrat en suspension dans l'acide fort.

**15.** Procédé selon l'une des revendications 1 à 14 caractérisé par le fait que le 3-alkoxy-4-méthoxybenzaldéhyde est préparé par O-méthylation d'un 3-alkoxy-4-hydroxybenzaldéhyde présentant un groupe alkoxy ayant au moins deux atomes de carbone.

**16.** Procédé selon la revendication 15 caractérisé par le fait que le 3-alkoxy-4-hydroxybenzaldéhyde répond à la formule (II) suivante :

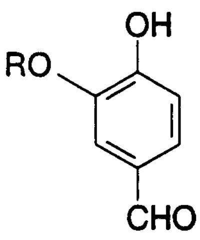

(II)

dans ladite formule (II), R représente un radical alkyle ou cycloalkyle ayant au moins 2 atomes de carbone.

**17.** Procédé selon la revendication 16 caractérisé par le fait que le 3-alkoxy-4-hydroxybenzaldéhyde répond à la formule (II) dans laquelle le radical R est un radical alkyle ou cycloalkyle dont le nombre d'atomes de carbone varie entre 2 et 8 atomes de carbone et plus préférentiellement un radical éthyle, propyle, isopropyle, n-butyle, isobutyle, sec-butyle, tert-butyle.

**18.** Procédé selon l'une des revendications 16 et 17 caractérisé par le fait que le 3-alkoxy-4-hydroxybenzaldéhyde est le 3-éthoxy-4-hydroxybenzaldéhyde, le 3-n-propoxy-4-hydroxybenzaldéhyde, le 3-isopropoxy-4-hydroxybenzaldéhyde, le 3-n-butoxy-4-hydroxybenzaldéhyde, le 3-isobutoxy-4-hydroxybenzaldéhyde, le 3-sec-butoxy-4-hydroxybenzaldéhyde, le 3-tert-butoxy-4-hydroxybenzaldéhyde.

**19.** Procédé selon l'une des revendications 15 à 18 caractérisé par le fait que - l'agent alkylant est un halogénure de méthyle, de préférence le chlorure de méthyle, le bromure de méthyle, le diméthylsulfate, le p-toluènesulfonate de méthyle, le méthanesulfonate de méthyle et plus préférentiellement, le chlorure de méthyle et le diméthylsulfate.

**20.** Procédé selon l'une des revendications 15 à 19 caractérisé par le fait que la réaction de O-méthylation est conduite à un pH compris entre 8 et 10 et de préférence, supérieur à 9, pH obtenu par addition si nécessaire d'une base, de préférence, une solution aqueuse d'un hydroxyde, de carbonate ou d'hydrogénocarbonate de métal alcalin, et plus

particulièrement la soude.

21. Procédé selon l'une des revendications 15 à 20 caractérisé par le fait que le rapport molaire agent d'alkylation/3-alkoxy-4-hydroxybenzaldéhyde varie entre 1,0 à 2,0 et, de préférence, entre 1,1 et 1,5.

22. Procédé selon l'une des revendications 15 à 21 caractérisé par le fait que la température de la réaction de O-méthylation est comprise entre 60°C et 100°C, de préférence, entre 80°C et 95°C.

23. Procédé selon l'une des revendications 15 à 22 caractérisé par le fait que l'on introduit d'abord le 3-alkoxy-4-hydroxybenzaldéhyde, la solution basique, puis l'on amène le mélange à la température réactionnelle, que l'on ajoute l'agent d'alkylation et éventuellement la base si nécessaire et que l'on récupère une phase organique comprenant le 3-alkoxy-4-méthoxybenzaldéhyde qui est ensuite séparé.

24. Procédé selon l'une des revendications 1 à 23 caractérisé par le fait que les deux étapes réactionnelles sont conduites sous une atmosphère de gaz inerte, de préférence un gaz rare ou l'azote.

**Claims**

1. Process for the preparation of isovanillin, characterized in that it consists in carrying out the dealkylation, using a strong acid, selectively in the 3-position, of a 3-alkoxy-4-methoxybenzaldehyde in which the alkoxy group has at least two carbon atoms.

2. The process according to claim 1, characterized in that the 3-alkoxy-4-methoxybenzaldehyde corresponds to the following formula (I):

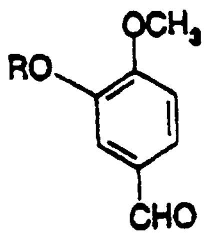
(I)

in the said formula (I), R represents an alkyl or cycloalkyl radical having at least 2 carbon atoms.

3. Process according to either of claims 1 and 2, characterized in that the 3-alkoxy-4-methoxybenzaldehyde corresponds to the formula (I) in which the radical R is an alkyl or cycloalkyl radical having a number of carbon atoms which ranges between 2 and 8, and more preferably an ethyl, propyl, isopropyl, n-butyl, isobutyl, sec-butyl or tert-butyl radical.

4. Process according to one of claims 1 to 3, characterized in that the 3-alkoxy-4-methoxybenzaldehyde is 3-ethoxy-4-methoxybenzaldehyde, 3-n-propoxy-4-methoxybenzaldehyde, 3-isopropoxy-4-methoxybenzaldehyde, 3-n-butoxy-4-methoxybenzaldehyde, 3-isobutoxy-4-methoxybenzaldehyde, 3-sec-butoxy-4-methoxybenzaldehyde and 3-tert-butoxy-4-methoxybenzaldehyde.

5. Process according to one of claims 1 to 4, characterized in that the strong acid has a pKa of less than or equal to 3.

6. Process according to claim 5, characterized in that the strong acid used is chosen from: halogenated acids such as hydrochloric acid, hydrobromic acid or hydriodic acid; halogenated or non-halogenated oxyacids, preferably sulphuric acid, pyrosulphuric acid or perchloric acid; halogenated or non-halogenated sulphonic acids, preferably fluorosulphonic acid, chlorosulphonic acid, trifluoromethanesulphonic acid, methanesulphonic acid, ethanesulphonic acid, ethanedisulphonic acid, benzenesulphonic acid, benzenedisulphonic acids, toluenesulphonic acids, naphthalenesulphonic acids and naphthalenedisulphonic acids.

7. Process according to either of claims 5 and 6, characterized in that the strong acid used is hydrochloric acid, hydrobromic acid or sulphuric acid.

**8.** Process according to one of claims 5 to 7, characterized in that the concentration of strong acid ranges between 30 and 50% by weight when the strong acid is hydrochloric acid or hydrobromic acid.

**9.** Process according to one of claims 5 to 8, characterized in that the concentration of strong acid ranges between 80 and 100% by weight, preferably between 90 and 99% by weight, and even more preferably between 95 and 99% by weight, when the strong acid used is sulphuric acid.

**10.** Process according to one of claims 5 to 9, characterized in that the ratio between the number of moles of strong acid used and the number of moles of 3-alkoxy-4-methoxybenzaldehyde ranges between 2 and 80, preferably between 3 and 15 and even more preferably between 4 and 10.

**11.** Process according to one of claims 1 to 10, characterized in that the reaction temperature ranges between 0°C and 150°C.

**12.** Process according to claim 11, characterized in that the reaction temperature ranges: in the case of sulphuric acid, between 50 and 90°C, preferably between 60°C and 80°C when the alkyl group to be removed is a linear alkyl group, and between 0°C and 40°C, preferably between 0°C and 30°C, when it is a branched alkyl group; in the case of hydrochloric acid or hydrobromic acid, between 100°C and 150°C, preferably between 100°C and 130°C when the alkyl group to be removed is a linear alkyl group, and between 50°C and 100°C, preferably between 60°C and 80°C, when it is a branched alkyl group.

**13.** Process according to one of claims 1 to 12, characterized in that the solution of strong acid is introduced into the 3-alkoxy-4-methoxybenzaldehyde, or vice versa.

**14.** Process according to one of claims 1 to 13, characterized in that the molten 3-alkoxy-4-methoxybenzaldehyde is introduced into the strong acid, or the substrate is added in suspension in the strong acid.

**15.** Process according to one of claims 1 to 14, characterized in that the 3-alkoxy-4-methoxybenzaldehyde is prepared by O-methylating a 3-alkoxy-4-hydroxybenzaldehyde having an alkoxy group which has at least two carbon atoms.

**16.** Process according to claim 15, characterized in that the 3-alkoxy-4-hydroxybenzaldedhyde corresponds to the following formula (II):

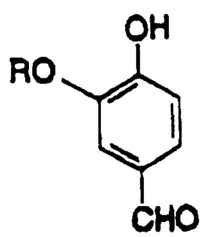

(II)

in the said formula (II), R represents an alkyl or cycloalkyl radical having at least 2 carbon atoms.

**17.** Process according to claim 16, characterized in that the 3-alkoxy-4-hydroxybenzaldehyde corresponds to the formula (II) in which the radical R is an alkyl or cycloalkyl radical having a number of carbon atoms which ranges between 2 and 8, and preferably an ethyl, propyl, isopropyl, n-butyl, isobutyl, sec-butyl or tert-butyl radical.

**18.** Process according to either of claims 16 and 17, characterized in that the 3-alkoxy-4-hydroxybenzaldehyde is 3-ethoxy-4-hydroxybenzaldehyde, 3-n-propoxy-4-hydroxybenzaldehyde, 3-isopropoxy-4-hydroxybenzaldehyde, 3-n-butoxy-4-hydroxybenzaldehyde, 3-isobutoxy-4-hydroxybenzaldehyde, 3-sec-butoxy-4-hydroxybenzaldehyde and 3-tert-butoxy-4-hydroxybenzaldehyde.

**19.** Process according to one of claims 15 to 18, characterized in that the alkylating agent is a methyl halide, preferably methyl chloride or methyl bromide, dimethyl sulphate, methyl p-toluenesulphonate or methyl methanesulphonate, and more preferably methyl chloride or dimethyl sulphate.

**20.** Process according to one of claims 15 to 19, characterized in that the O-methylation reaction is performed at a pH of between 8 and 10 and, preferably, above 9, this pH being obtained by addition, if necessary, of a base, preferably an aqueous solution of an alkali metal hydrogen carbonate, carbonate or hydroxide, and more particularly sodium hydroxide.

**21.** Process according to one of claims 15 to 20, characterized in that the alkylating agent/3-alkoxy-4-hydroxybenzaldehyde molar ratio ranges between 1.0 and 2.0 and, preferably, between 1.1 and 1.5.

**22.** Process according to one of claims 15 to 21, characterized in that the temperature of the O-methylation reaction is between 60°C and 100°C, preferably between 80°C and 95°C.

**23.** Process according to one of claims 15 to 22, characterized in that, firstly, the 3-alkoxy-4-hydroxybenzaldehyde and the basic solution are introduced and the mixture is then brought to the reaction temperature, in that the alkylating agent is added and optionally the base, if required, and in that an organic phase comprising the 3-alkoxy-4-methoxybenzaldehyde is recovered and is then separated out.

**24.** Process according to one of claims 1 to 23, characterized in that the two reaction steps are performed under an atmosphere of inert gas, preferably a rare gas or nitrogen.

**Patentansprüche**

**1.** Verfahren zur Herstellung von Isovanillin, dadurch gekennzeichnet, daß ein 3-Alkoxy 4-methoxybenzaldehyd, in dem die Alkoxy-Gruppe mindestens zwei Kohlenstoffatome hat, mit Hilfe einer starken Säure selektiv in 3-Stellung entalkyliert wird.

**2.** Verfahren nach Anspruch 1, dadurch gekennzeichnet, daß der 3-Alkoxy 4-methoxybenzaldehyd der folgenden Formel (I) entspricht.

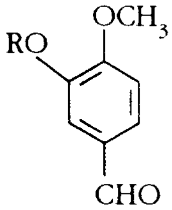

(I)

in der R einen Alkyl- oder Cycloalkylrest mit mindestens zwei Kohlenstoffatomen bedeutet.

**3.** Verfahren nach einem der Ansprüche 1 oder 2, dadurch gekennzeichnet, daß der 3-Alkoxy-4-methoxybenzaldehyd die Formel (I) hat, in der R einen Alkyl- oder Cycloalkylrest mit 2 und 8 Kohlenstoffatomen, und vorzugsweise einen Ethyl-, Propyl-, Isopropyl-, n-Butyl-, Isobutyl-, sec.-Butyl- oder tert.-Butylrest bedeutet.

**4.** Verfahren nach einem der Ansprüche 1 bis 3, dadurch gekennzeichnet, daß der 3-Alkoxy-4-methoxybenzaldehyd 3-Ethoxy-4-methoxybenzaldehyd, 3-n-Propoxy-4-methoxybenzaldehyd, 3-Isopropory-4-methoxyhenzaldehyd, 3-n-Butoxy-4-methoxyhenzaldehyd, 3-Isobutoxy-4-methoxyhenzaldehyd, 3-sec.-Butoxy-4-methoxybenzaldehyd oder 3-tert.-Butory-4-methoxybenzaldehvd ist.

**5.** Verfahren nach einem der Ansprüche 1 bis 4, dadurch gekennzeichnet, daß die starke Säure einen pKa-Wert von kleiner oder gleich 3 hat.

**6.** Verfahren nach Anspruch 5, dadurch gekennzeichnet, daß die eingesetzte starke Säure unter folgenden ausgewählt ist: den Halogensäuren, wie z.B. Chlorwasserstoff, Bromwasserstoff, Jodwasserstoff; den gegebenenfalls halogenierten Sauerstoffsäuren, vorzugsweise Schwefelsäure, Dischwefelsäure, Perchlorsäure; den gegebenenfalls halogenierten Sulfonsäuren, vorzugsweise Fluorschwefelsäure, Chlorschwefelsäure, Trifluorrnethansulfonsäure, Methansulfonsäure, Ethansulfonsäure, Ethandisulfonsäure, Benzolsulfonsäure, den Benzoldisulfonsäuren, den Toluolsulfonsäuren, den Naphthalinsulfonsäuren und den Naphthalindisulfonsäuren.

7. Verfahren nach einem der Ansprüche 5 oder 6, dadurch gekennzeichnet, daß die eingesetzte starke Säure Chlorwasserstoff, Bromwasserstoff oder Schwefelsäure ist.

8. Verfahen nach einem der Ansprüche 5 bis 7, dadurch gekennzeichnet, daß die Konzentration der starken Säure zwischen 30 und 50 Gew.-% schwankt, wenn die starke Säure Chlorwasserstoff oder Bromwasserstoff ist.

9. Verfahren nach einem der Ansprüche 5 bis 8, dadurch gekennzeichnet, daß die Konzentration der starken Säure zwischen 80 und 100 Gew.-%, bevorzugt zwischen 90 und 99 Gew.-% und speziell zwischen 95 und 99 Gew.% schwankt, wenn die starke Säure Schwefelsäure ist

10. Verfahren nach einem der Ansprüche 5 bis 9, dadurch gekennzeichnet, daß das Verhältnis zwischen der Molzahl der verwendeten starken Säure und der Molzahl des 3-Alkoxy-4-methoxybenzaldehyds zwischen 2 und 80, bevorzugt zwischen 3 und 15 und speziell zwischen 4 und 10 schwankt.

11. Verfahren nach einem der Ansprüche 1 bis 10, dadurch gekennzeichnet, daß die Reaktionstemperatur zwischen 0°C und 150 °C schwankt.

12. Verfahren nach Anspruch 11, dadurch gekennzeichnet, daß die Reaktionstemperatur wie folgt schwankt: Bei Anwendung von Schwefelsäure zwischen 50 und 90 °C, vorzugsweise zwischen 60°C und 80 °C, beträgt, wenn die abzuspaltende Alkylgruppe eine lineare Alkylgruppe ist und zwischen 0 °C und 40 °C, vorzugsweise zwischen 0 °C und 30 °C, wenn es sich um eine verzweigte Alkylgruppe handelt; bei Anwendung von Chlorwasserstoff oder Bromwasserstoff zwischen 100 °C und 150 °C, vorzugsweise zwischen 100 °C und 130 °C, wenn die abzuspaltende Alkylgruppe eine lineare Alkylgruppe ist und zwischen 50 °C und 100 °C, vorzugsweise zwischen 60 °C und 80 °C, wenn es sich um eine verzweigte Alkylgruppe handelt.

13. Verfahren nach einem der Ansprüche 1 bis 12, dadurch gekennzeichnet, daß man die Lösung der starken Säure dem 3-Alkoxy-4-methoxybenzaldehyd gibt oder umgekehrt.

14. Verfahren nach einem der Ansprüche 1 bis 13, dadurch gekennzeichnet, daß man den geschmolzenen 3-Alkoxy-4-methoxybenzaldehyd in die starke Säure einträgt oder das Substrat als Suspension in starker Säure zugibt.

15. Verfahren nach einem der Ansprüche 1 bis 14, dadurch gekennzeichnet, daß der 3-Alkoxy-4-methoxybenzaldehyd durch O-Methylierung von einem eine Alkoxygruppe von mindestens zwei Kohlenstoffatomen aufweisenden 3-Alkoxy-4-hydroxybenzaldehyd hergestellt wird.

16. Verfahren nach Anspruch 15, dadurch gekennzeichnet, daß 3-Alkoxy-4-hydroxybenzaldehyd der nachfolgenden Formel (II) entspricht:

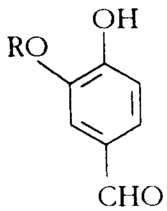

(II)

in der R einen Alkyl- oder Cycloalkylrest mit mindestens zwei Kohlenstoffatomen bedeutet.

17. Verfahren nach Anspruch 16, dadurch gekennzeichnet, daß der 3-Alkoxy-4-hydroxybenzaldehyd der Formel (II) genügt, in der R ein Alkyl- oder Cycloalkylrest mit 2 bis 8 Kohlenstoffatomen, vorzugsweise ein Ethyl-, Propyl-, Isopropyl-, n-Butyl-, Isobutyl-, sec.-Butyl- oder tert.-Butylrest ist.

18. Verfahren nach einem der Ansprüche 16 oder 17, dadurch gekennzeichnet, daß 3-Alkoxy-4-hydroxyberzaldehyd 3-Ethoxy-4-hydroxybenzaldehyd, 3-n-Propoxy-4-hydroxybenzaldehyd, 3-Isopropoxy-4-hydroxybenzaldehyd, 3-n-Butoxy-4-hydroxybenzaldehyd, 3-Isobutoxy-4-hydroxybenzaldehyd, 3-sec.-Butoxy-4-hydroxybenzaldehyd oder 3-tert.-Butoxy-4-hydroxybenzaldehyd ist.

**19.** Verfahren nach einem der Ansprüche 15 bis 18, dadurch gekennzeichnet, daß das Alkylierungsmittel ein Methylhalogenid, vorzugsweise Methylchlorid oder -bromid, ferner Dimethylsulfat, Methyl-p-toluolsulfonat, Methylmethansulfonat und insbesondere Methylchlorid und Dimethylsulfat ist.

**20.** Verfahren nach einem der Ansprüche 15 bis 19, dadurch gekennzeichnet, daß die O-Methylierungsreaktion bei einem pH-Wert zwischen 8 und 10 und vorzugsweise oberhalb von 9 durchgeführt wird, der erforderlichenfalls durch Zugabe einer Base, vorzugsweise einer wäßrigen Lösung eines Hydroxyds, Carbonats oder Hydrogencarbonats eines Alkalimetalls und insbesondere von Natrohlauge erhalten wird.

**21.** Verfahren nach einem der Ansprüche 15 bis 20, dadurch gekennzeichnet, daß das molare Verhältnis zwischen dem Alkylierungsmittel und 3-Alkoxy-4-hydroxybenzaldehyd zwischen 1,0 und 2,0 und vorzugsweise zwischen 1,1 und 1,5 schwankt.

**22.** Verfahren nach einem der Ansprüche 15 bis 21, dadurch gekennzeichnet, daß die Temperatur der O-Methylierungsreaktion zwischen 60 °C und 100 °C, vorzugsweise zwischen 80 °C und 95 °C betragt.

**23.** Verfahren nach einem der Ansprüche 15 bis 22, dadurch gekennzeichnet, daß man zunächst 3-Alkoxy-4-hydroxybenzaldehyd und die basische Lösung einträgt, danach die Mischung auf die Reaktionstenlperatur bringt, das Alkylierungsmittel und gegebenenfalls, wenn erforderlich, die Base zufügt und eine organische Phase gewinnt, welche 3-Alkoxy-4-methoxybenzaldehyd enthält, das anschließend abgetrennt wird.

**24.** Verfahren nach einem der Ansprüche 1 bis 23, dadurch gekennzeichnet, daß die zwei Reaktionsstufen unter einem Inertgas, vorzugsweise einem Edelgas oder Stickstoff, durchgeführt werden.